# EUROPEAN PATENT APPLICATION

(11) **EP 3 730 039 A1**
(43) Date of publication of application: **28.10.2020**
(21) Application number: 17935446.9
(22) Date of filing: 26.12.2017
(51) Int. Cl.: A61B 5/00

(54) **AIRWAY OCT CATHETER**

(30) Priority: 18.12.2017 CN 201711368241
(71) Applicant: Guangzhou Winstar Medical Technology Company Limited, Guangzhou, Guangdong 510663 (CN)
(72) Inventor: DU, Ningyi, Guangzhou, Guangdong 510663 (CN); LI, Bailing, Guangzhou, Guangdong 510663 (CN); SONG, Liyan, Guangzhou, Guangdong 510663 (CN); LIANG, Weiliang, Guangzhou, Guangdong 510663 (CN); GAO, Jun, Guangzhou, Guangdong 510663 (CN)
(74) Representative: Sun, Yiming
(86) International application number: PCT/CN2017/118609
(87) International publication number: WO 2019/119483

(57) **Abstract**

The present invention provides an OCT catheter for the respiratory tract, which includes an optical fiber connecting part, where the optical fiber connecting part is connected to an optical fiber, and optical signals are transmitted to the optical fiber through the optical fiber connecting part; the optical fiber connecting part includes a connector, a fixing head, a sealing assembly, a guide wire assembly and an optical fiber interface; the guide wire assembly is sleeved outside the optical fiber, the optical fiber is connected to the optical fiber interface, an end of the guide wire assembly is sleeved inside the optical fiber interface, the optical fiber interface is connected to an optical fiber coupler of a driving device, the optical fiber coupler is sleeved inside the connector, the connector is sleeved inside the fixing head, an end of the fixing head is sleeved inside the sealing assembly, and the guide wire assembly sequentially passes through the connector, the fixing head and the sealing assembly. The OCT catheter for the respiratory tract according to the present invention is connected to the SC-type optical fiber interface through the SC-SC optical fiber coupler of a driving module. By adopting the guide wire with a small diameter, the medical OCT catheter can be applied to higher-generation bronchi, thereby expanding the application range and facilitating popularization.

## Description

### TECHNICAL FIELD

The present invention relates to the technical field of optical imaging, and in particular, to an optical coherence tomography (OCT) catheter for the respiratory tract.

### BACKGROUND

OCT is a high-resolution imaging technology which is based on the principle of optical low-coherence reflectometry and combined with the confocal microscopy technology. This technology can be used to detect backscattering wave echo time delay and echo intensity signals of different depth layers of biological tissues to incident weak coherent light, and a two-dimensional or three-dimensional high-resolution microstructure of a sample is obtained by scanning, so that an in vivo nondestructive tomographic image of the tested sample is obtained.

Tissue images of lumens can be effectively obtained by using an OCT imaging technology for 360° scanning detection. However, existing OCT catheter products cannot enter narrow lumens such as the higher-generation bronchi because of their large sizes. This limits the interventional diagnosis and treatment of these lesions and the application and popularization of the OCT technology.

### SUMMARY

In order to overcome the shortcomings of the prior art, an objective of the present invention is to provide an OCT catheter for the respiratory tract, which is connected to an SC-type optical fiber interface through an SC-SC optical fiber coupler of a driving module. By adopting a guide wire with a small diameter, the medical OCT catheter can be applied to higher-generation bronchi, thereby expanding the application range and facilitating popularization.

The present invention provides the OCT catheter for the respiratory tract, which includes an optical fiber connecting part, where the optical fiber connecting part is connected to an optical fiber, and optical signals are transmitted to the optical fiber through the optical fiber connecting part; the optical fiber connecting part includes a connector, a fixing head, a sealing assembly, a guide wire assembly and an optical fiber interface, where the guide wire assembly is sleeved outside the optical fiber, the optical fiber is connected to the optical fiber interface, an end of the guide wire assembly is sleeved inside the optical fiber interface, the optical fiber interface is connected to an optical fiber coupler of a driving device, the optical fiber coupler is sleeved inside the connector, the connector is sleeved inside the fixing head, an end of the fixing head is sleeved inside the sealing assembly, and the guide wire assembly sequentially passes through the connector, the fixing head and the sealing assembly.

Further, the sealing assembly includes a sealing head, a tail tube and a protective leather sleeve, where a first end of the sealing head is provided with an annular groove, an end of the fixing head is sleeved inside the annular groove, a second end of the sealing head is provided with a first axial groove and a second axial groove, the tail tube is sleeved outside the first axial groove, one end of the protective leather sleeve is sleeved outside the second axial groove, the other end of the protective leather sleeve extends out of the tail tube, and the guide wire assembly sequentially passes through the sealing head, the tail tube and the protective leather sleeve.

Further, the sealing assembly further includes sealing rings and a joint steel tube, where an end of the joint steel tube is sleeved inside the optical fiber interface, the guide wire assembly is sleeved inside the other end of the joint steel tube, the sealing rings are installed on the joint steel tube, and the sealing rings are located at a port where the fixing head is connected to the sealing head.

Further, the guide wire assembly includes a guide wire, a guide wire fixing tube, a collimator and a positioning tube, where an end of the guide wire fixing tube is sleeved inside the joint steel tube, one end of the guide wire is sleeved inside the other end of the guide wire fixing tube, the other end of the guide wire is connected to the collimator, the positioning tube is sleeved outside the collimator, and the guide wire is sleeved outside the optical fiber.

Further, the guide wire assembly includes an elastic nylon catheter, where the elastic nylon catheter is sleeved outside the guide wire, and the diameter of the guide wire assembly is formed by sleeving the guide wire and the optical fiber with the elastic nylon catheter.

Further, the connector is provided with an annular groove, the fixing head is provided with a plurality of positioning through holes, the annular groove corresponds to the positioning through holes, and a locking piece passes through the positioning through holes and the annular groove to limit an axial position of the fixing head.

Further, the optical fiber interface is specifically an SC-type optical fiber interface, and the optical fiber coupler is specifically an SC-SC optical fiber coupler.

Further, the guide wire assembly has a diameter of 1.7-1.8 mm.

Further, there are at least two sealing rings.

Further, the positioning tube is made of stainless steel, and the collimator and the positioning tube are wrapped in the elastic nylon catheter.

Compared with the prior art, the present invention has the following beneficial effects.

The present invention provides an OCT catheter for the respiratory tract, which includes an optical fiber connecting part, where the optical fiber connecting part is connected to an optical fiber, and optical signals are transmitted to the optical fiber through the optical fiber connecting part; the optical fiber connecting part includes a connector, a fixing head, a sealing assembly, a guide wire assembly and an optical fiber interface; the guide wire assembly is sleeved outside the optical fiber, the optical fiber is connected to the optical fiber interface, an end of the guide wire assembly is sleeved inside the optical fiber interface, the optical fiber interface is connected to an optical fiber coupler of a driving device, the optical fiber coupler is sleeved inside the connector, the connector is sleeved inside the fixing head, an end of the fixing head is sleeved inside the sealing assembly, and the guide wire assembly sequentially passes through the connector, the fixing head and the sealing assembly. The OCT catheter for the respiratory tract according to the present invention is connected to the SC-type optical fiber interface through the SC-SC optical fiber coupler of a driving module. By adopting the guide wire with a small diameter, the medical OCT catheter can be applied to higher-generation bronchi, thereby expanding the application range and facilitating popularization.

The above description is only an overview of the technical solutions of the present invention. In order that the technical means of the present invention can be more clearly understood and implemented in accordance with the contents of the specification, the preferred embodiments of the present invention will be described in detail below with reference to the accompanying drawings. The specific embodiments of the present invention are given in detail by the following embodiments and accompanying drawings thereof.

### BRIEF DESCRIPTION OF DRAWINGS

Accompanying drawings described herein are intended to provide a further understanding of the present invention, which constitutes a part of the present application. Schematic embodiments of the present invention and the description thereof are intended to explain the present invention and do not constitute an undue limitation on the present invention. In the accompanying drawings:
FIG. 1 is a schematic diagram of an OCT catheter for the respiratory tract according to the present invention;
FIG. 2 is a partial sectional view of an OCT catheter for the respiratory tract according to the present invention;
FIG. 3 is a view of a first part of an OCT catheter for the respiratory tract according to the present invention; and
FIG. 4 is a view of a second part of an OCT catheter for the respiratory tract according to the present invention.

In the figures: 1. medical OCT catheter, 11. optical fiber connecting part, 111. connector, 1111. annular groove of the connector, 112. fixing head, 1121. positioning through hole, 113. sealing assembly, 1131. sealing head, 1132. tail tube, 1133, protective leather sleeve, 1134. sealing ring, 1135. joint steel tube, 114. guide wire assembly, 1141. guide wire, 1142. guide wire fixing tube, 1143. elastic nylon catheter, 1144. collimator, 1145. positioning tube, 115. optical fiber interface, 12. optical fiber, 2. optical fiber coupler.

### DESCRIPTION OF EMBODIMENTS

The present invention will be further described below with reference to the accompanying drawings and specific embodiments. It should be noted that, all embodiments described below or all the technical features can be arbitrarily combined to form new embodiments, provided that they do not conflict with each other.

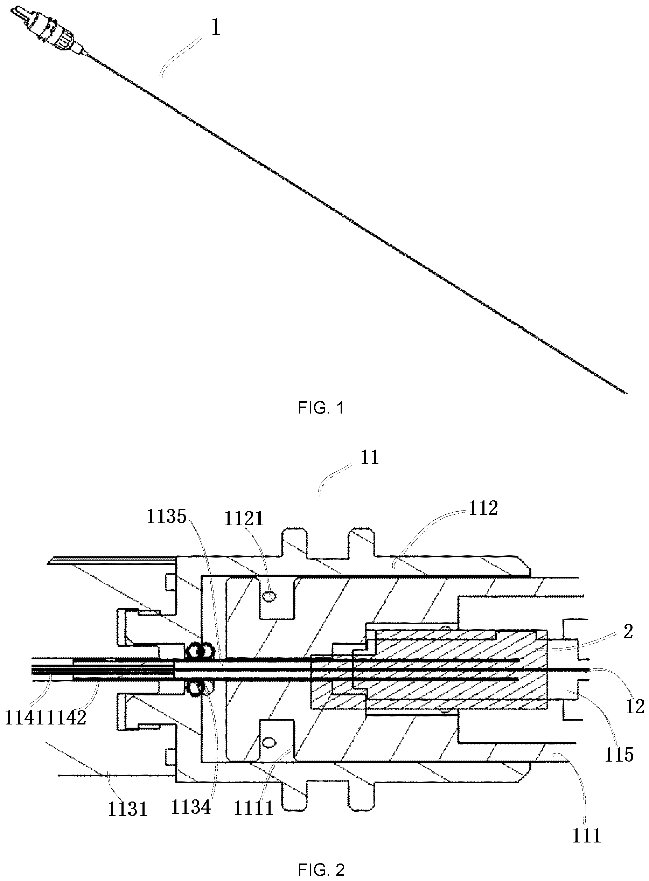

As shown in FIGs. 1-2, an OCT catheter 1 for the respiratory tract includes an optical fiber connecting part 11. The optical fiber connecting part 11 is connected to an optical fiber 12, and optical signals are transmitted to the optical fiber 12 through the optical fiber connecting part 11. The optical fiber connecting part 11 includes a connector 111, a fixing head 112, a sealing assembly 113, a guide wire assembly 114 and an optical fiber interface 115. The guide wire assembly 114 is sleeved outside the optical fiber 12, and the optical fiber 12 is connected to the optical fiber interface 115. An end of the guide wire assembly 114 is sleeved inside the optical fiber interface 115, the optical fiber interface 115 is connected to an optical fiber coupler 2 of a driving device, the optical fiber coupler 2 is sleeved inside the connector 111, the connector 111 is sleeved inside the fixing head 112, and an end of the fixing head 112 is sleeved inside the sealing assembly 113. The guide wire assembly 114 sequentially passes through the connector 111, the fixing head 112 and the sealing assembly 113. When the driving device rotates, the optical fiber coupler 2 of the driving device rotates. Because the optical fiber coupler 2 of the driving device is in interference fit with the connector 111, and the connector 111 is in interference fit with the guide wire assembly 114, the rotation of the optical fiber coupler 2 drives the connector 111 to rotate and further drives the guide wire assembly 114 to rotate.

As shown in FIGs. 2-3, preferably, the sealing assembly 113 includes a sealing head 1131, a tail tube 1132 and a protective leather sleeve 1133. A first end of the sealing head 1131 is provided with an annular groove, and an end of the fixing head 112 is sleeved inside the annular groove. A second end of the sealing head 1131 is provided with a first axial groove and a second axial groove. The tail tube 1132 is sleeved outside the first axial groove, one end of the protective leather sleeve 1133 is sleeved outside the second axial groove, and the other end of the protective leather sleeve 1133 extends out of the tail tube 1132. The guide wire assembly 114 sequentially passes through the sealing head 1131, the tail tube 1132 and the protective leather sleeve 1133. In this embodiment, the protective leather sleeve 1133 is conical, and the diameter of a first port of the protective leather sleeve 1133 is greater than that of a second port. The first port of the protective leather sleeve 1133 is sleeved outside the second axial groove at the second end of the sealing head 1131, and the second port of the protective leather sleeve 1133 extends out of the tail tube 1132.

As shown in FIGs. 2-3, preferably, the sealing assembly 113 further includes sealing rings 1134 and a joint steel tube 1135. An end of the joint steel tube 1135 is sleeved inside the optical fiber interface 115, the guide wire assembly 114 is sleeved inside the other end of the joint steel tube 1135, the sealing rings 1134 are installed on the joint steel tube 1135, and the sealing rings 1134 are located at a port where the fixing head 112 is connected to the sealing head 1131. Preferably, there are at least two sealing rings 1134. In this embodiment, there are two sealing rings 1134, and the sealing rings 1134 are used for preventing internal oil leakage.

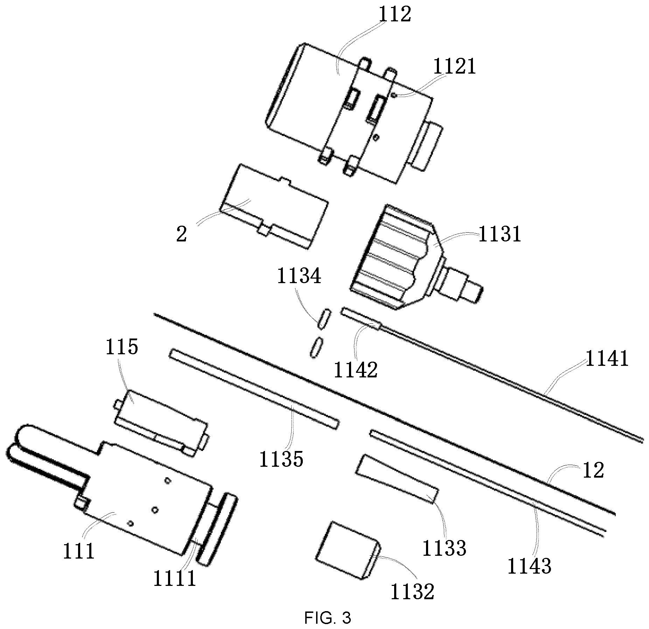

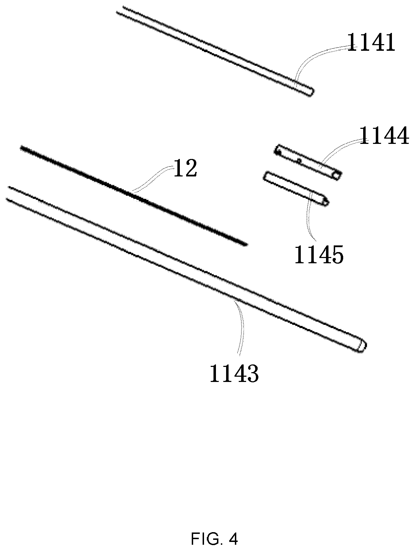

As shown in FIGs. 3-4, preferably, the guide wire assembly 114 includes a guide wire 1141, a guide wire fixing tube 1142, a collimator 1144 and a positioning tube 1145. An end of the guide wire fixing tube 1142 is sleeved inside the joint steel tube 1135, one end of the guide wire 1141 is sleeved inside the other end of the guide wire fixing tube 1142, the other end of the guide wire 1141 is connected to the collimator 1144, and the positioning tube 1145 is sleeved outside the collimator 1144. The guide wire 1141 is sleeved outside the optical fiber 12, and the guide wire 1141 is spiral. When the driving device rotates, the optical fiber coupler 2 of the driving device rotates. Because the optical fiber coupler 2 of the driving device is in interference fit with the connector 111,the connector 111 is in interference fit with the joint steel tube 1135, the joint steel tube 1135 is in interference fit with the guide wire fixing tube 1142, and the fixing tube 1142 is interference fit with the guide wire 1141, the rotation of the optical fiber coupler 2 drives the connector 111 to rotate; drives the joint steel tube 1135 to rotate; drives the guide wire fixing tube 1142 to rotate; and drives the guide wire 1141 to rotate. Because the guide wire 1141 is in clearance fit with the optical fiber 12, the optical fiber 12 does not move while the guide wire 1141 rotates.

As shown in FIG. 3, preferably, the guide wire assembly 114 further includes an elastic nylon catheter 1143. The elastic nylon catheter 1143 is sleeved outside the guide wire 1141. The diameter of the guide wire assembly 114 is formed by sleeving the guide wire 1141 and the optical fiber 12 with the elastic nylon catheter 1143. Preferably, the guide wire assembly 114 has a diameter of 1.7-1.8 mm. In this embodiment, the guide wire assembly 114 has a diameter of 1.77 mm, so that the medical OCT catheter can be applied to higher-generation bronchi. Preferably, the positioning tube 1145 is made of stainless steel, the collimator 1144 and the positioning tube 1145 are wrapped in the elastic nylon catheter 1143, and the elastic nylon catheter 1143 protects the guide wire assembly 114 and the optical fiber 12 from excessive bending.

As shown in FIGs. 2-3, preferably, the connector 111 is provided with an annular groove 1111, and the fixing head 112 is provided with a plurality of positioning through holes 1121. Every two positioning through holes 1121 are symmetrical with respect to the central axis of the fixing head 112. The annular groove 1111 corresponds to the positioning through holes 1121. A locking piece passes through the positioning through holes 1121 and the annular groove 1111 to limit an axial position of the fixing head 112. For example, a bolt penetrates through the positioning through hole 1121 at one side of the fixing head 112, passes through the annular groove 1111, and then penetrates out of the positioning through hole 1121 at the symmetrical side of the fixing head 112. The position of the annular groove 1111 is locked by the bolt to limit the fixing head 112, so that the fixing head 112 and the sealing head 1131 are fixedly connected with each other and kept still.

In an embodiment, preferably, the optical fiber interface 115 is specifically an SC-type optical fiber interface, and the SC-type optical fiber interface adopts push-pull connection. An inner contact of the SC-type optical fiber interface is a copper column which is small and light, can be installed fast and reliably at high density, and is low in insertion loss and high in return loss. The optical fiber coupler 2 is specifically an SC-SC optical fiber coupler, and the optical fiber 12 is a single-mode optical fiber. In the present invention, the medical OCT catheter has a reduced size by using the SC-type optical fiber interface and the SC-SC optical fiber coupler, so that the medical OCT catheter can be applied to higher-generation bronchi, thereby expanding the application range and facilitating popularization.

The present invention provides an OCT catheter for the respiratory tract, which includes an optical fiber connecting part, where the optical fiber connecting part is connected to an optical fiber, and optical signals are transmitted to the optical fiber through the optical fiber connecting part; the optical fiber connecting part includes a connector, a fixing head, a sealing assembly, a guide wire assembly and an optical fiber interface; the guide wire assembly is sleeved outside the optical fiber, the optical fiber is connected to the optical fiber interface, an end of the guide wire assembly is sleeved inside the optical fiber interface, the optical fiber interface is connected to an optical fiber coupler of a driving device, the optical fiber coupler is sleeved inside the connector, the connector is sleeved inside the fixing head, an end of the fixing head is sleeved inside the sealing assembly, and the guide wire assembly sequentially passes through the connector, the fixing head and the sealing assembly. The OCT catheter for the respiratory tract according to the present invention is connected to the SC-type optical fiber interface through the SC-SC optical fiber coupler of a driving module. By adopting the guide wire with a small diameter, the medical OCT catheter can be applied to higher-generation bronchi, thereby expanding the application range and facilitating popularization.

The above is only preferred embodiments of the present invention, and do not limit the present invention in any form. Those of ordinary skill in the art can smoothly implement the present invention as per the accompanying drawings of the specification and the above. Equivalent slight changes, modifications and evolutions made by those skilled in the art without departing from the scope of the technical solutions of the present invention by utilizing the technical contents disclosed above are all equivalent embodiments of the present invention. Besides, any equivalent changes, modifications and evolutions made to the foregoing embodiments according to the essential technology of the present invention still fall within the protection scope of the technical solutions of the present invention.

## Claims

1. An optical coherence tomography (OCT) catheter for the respiratory tract, comprising an optical fiber connecting part, wherein the optical fiber connecting part is connected to an optical fiber, and optical signals are transmitted to the optical fiber through the optical fiber connecting part; the optical fiber connecting part comprises a connector, a fixing head, a sealing assembly, a guide wire assembly and an optical fiber interface; the guide wire assembly is sleeved outside the optical fiber, the optical fiber is connected to the optical fiber interface, an end of the guide wire assembly is sleeved inside the optical fiber interface, the optical fiber interface is connected to an optical fiber coupler of a driving device, the optical fiber coupler is sleeved inside the connector, the connector is sleeved inside the fixing head, an end of the fixing head is sleeved inside the sealing assembly, and the guide wire assembly sequentially passes through the connector, the fixing head and the sealing assembly.

2. The OCT catheter for the respiratory tract according to claim 1, wherein the sealing assembly comprises a sealing head, a tail tube and a protective leather sleeve, a first end of the sealing head is provided with an annular groove, an end of the fixing head is sleeved inside the annular groove, a second end of the sealing head is provided with a first axial groove and a second axial groove, the tail tube is sleeved outside the first axial groove, one end of the protective leather sleeve is sleeved outside the second axial groove, the other end of the protective leather sleeve extends out of the tail tube, and the guide wire assembly sequentially passes through the sealing head, the tail tube and the protective leather sleeve.

3. The OCT catheter for the respiratory tract according to claim 2, wherein the sealing assembly further comprises sealing rings and a joint steel tube, an end of the joint steel tube is sleeved inside the optical fiber interface, the guide wire assembly is sleeved inside the other end of the joint steel tube, the sealing rings are installed on the joint steel tube, and the sealing rings are located at a port where the fixing head is connected to the sealing head.

4. The OCT catheter for the respiratory tract according to claim 3, wherein there are at least two sealing rings.

5. The OCT catheter for the respiratory tract according to claim 3, wherein the guide wire assembly comprises a guide wire, a guide wire fixing tube, a collimator and a positioning tube, an end of the guide wire fixing tube is sleeved inside the joint steel tube, one end of the guide wire is sleeved inside the other end of the guide wire fixing tube, the other end of the guide wire is connected to the collimator, the positioning tube is sleeved outside the collimator, and the guide wire is sleeved outside the optical fiber.

6. The OCT catheter for the respiratory tract according to claim 5, wherein the guide wire assembly further comprises an elastic nylon catheter, the elastic nylon catheter is sleeved outside the guide wire, and the diameter of the guide wire assembly is formed by sleeving the guide wire and the optical fiber with the elastic nylon catheter.

7. The OCT catheter for the respiratory tract according to claim 6, wherein the guide wire assembly has a diameter of 1.7-1.8 mm.

8. The OCT catheter for the respiratory tract according to claim 6, wherein the positioning tube is made of stainless steel, and the collimator and the positioning tube are wrapped in the elastic nylon catheter.

9. The OCT catheter for the respiratory tract according to claim 1, wherein the connector is provided with an annular groove, the fixing head is provided with a plurality of positioning through holes, the annular groove corresponds to the positioning through holes, and a locking piece passes through the positioning through holes and the annular groove to limit an axial position of the fixing head.

10. The OCT catheter for the respiratory tract according to claim 1, wherein the optical fiber interface is specifically an SC-type optical fiber interface, and the optical fiber coupler is specifically an SC-SC optical fiber coupler.
